# EUROPEAN PATENT SPECIFICATION

(11) **EP 1 100 892 B1**
(45) Date of publication and mention of the grant of the patent: **09.04.2008**
(21) Application number: 99935916.9
(22) Date of filing: 24.07.1999
(51) Int. Cl.: C12N 15/11, C12Q 1/68, A61P 35/00, A61K 48/00

(54) **USE OF PROHIBITIN RNA IN TREATMENT OF CANCER**
VERWENDUNG VON PROHIBITIN-RNS IN KREBSBEHANDLUNG
UTILISATION D'ARN DE PROHIBITINE DANS LE TRAITEMENT DU CANCER

(30) Priority: 24.07.1998 US 93977 P
(43) Date of publication of application: 23.05.2001
(73) Proprietor: Oklahoma Medical Research Foundation, Oklahoma City, OK 73104 (US)
(72) Inventor: JUPE, Eldon, R., Norman, OK 73071 (US)
(74) Representative: Dehmel, Albrecht
(86) International application number: PCT/US1999/016840
(87) International publication number: WO 2000/005359

(56) References cited:
- WO-A-96/40919
- WO-A-98/20167
- US-A- 5 658 792
- JUPE E R ET AL: "THE 3' UNTRANSLATED REGION OF PROHIBITIN AND CELLULAR IMMORTALIZATION" EXPERIMENTAL CELL RESEARCH,US,SAN DIEGO, CA, vol. 224, no. 1, 10 April 1996 (1996-04-10), page 128-135 XP000609312 ISSN: 0014-4827 cited in the application
- JUPE, E.R. ET AL.: "The prohibitin 3'UTR as a susceptibility marker and therapeutic in breast cancer" PROCEEDINGS OF THE AMERICAN ASSOCIATION FOR CANCER RESEARCH, vol. 40, March 1999 (1999-03), page 481 XP000857345

## Description

### TECHNICAL FIELD OF INVENTION

This invention relates generally to use of single-stranded oligonucleotides for the treatment of cancer and particularly to the use of a portion of a 3'UTR from a prohibitin gene (DNA) or a transcript thereof (RNA) for inhibiting the growth of cancer cells in animals.

### BACKGROUND OF THE INVENTION

Division of normal cells is controlled by complex interactions between factors telling the cell to divide and other factors telling the cell to stop dividing. Controlling the steps of cell division, collectively known as the cell cycle, is a complex interplay between promoter (oncogenes) and suppressor genes.

Cancer is a disease resulting from uncontrolled cell division caused by mutations in promoter (oncogenes) and/or suppressor genes: In cancer cells, oncogene products are over expressed and tumor suppressor gene products are lost. Initial evidence for the existence of tumor suppressor genes emerged from studies of chromosomal deletions in familial cancer syndromes. Deletions in these same regions are also often observed in sporadic cancers. The restoration of a single copy of a missing or altered tumor suppressor gene will often re-establish control over the growth of cancerous cell lines and suppress tumor formation in animal cancer models.

Many genes and their products with positive effects on cell proliferation such as growth factors and their cognate receptors, transcription factors, and cyclins and cyclin dependent kinases have been identified and extensively studied. These represent oncogenes whose aberrant overexpression leads to uncontrolled growth. Genes involved in cell cycle arrest have been more difficult to isolate and characterize because of their recessive nature. These tumor suppressors or negative regulators of cell proliferation exhibit loss of function in tumors.

Tumor suppressor genes code for negative regulators that suppress the proliferation of cells and are of immense interest because of their importance in understanding normal and cancerous cell growth. The prohibitin 3' untranslated region (3' UTR) falls into a major category of tumor suppressors that are inhibitors of DNA synthesis. The cDNA coding for prohibitin was originally identified and cloned in a screen to discover senescence regulating mRNAs highly expressed in normal compared to regenerating rat liver. (McClung, et al. 1989. "Isolation of a cDNA that hybrid selects antiproliferative mRNA from rat liver," Biochem Biophys Res Comm 164:1316-1322; and Nuell, et al. 1991. "Prohibitin, an evolutionarily conserved intracellular protein that blocks DNA synthesis in normal fibroblasts and HeLa cells," Mol Cell Bio 11:1372-1381).

The human prohibitin gene maps to chromosome 17 at q21 near *BRCA1,* and two alleles (designated "B" and "non-B") have been described. (Jupe, et al. 1995. "Prohibitin antiproliferative activity and a lack of heterozygosity in immortalized cell lines," Exp Cell Res 218:577-580; Jupe et al. 1996. "The 3' untranslated region of prohibitin and cellular immortalization," Exp Cell Res 224:128-135; Jupe, et al. 1996. "Prohibitin in breast cancer cell lines: loss of antiproliferative activity is linked to 3' untranslated region mutations," Cell Growth and Differentiation 7:871-878; and White, et al. 1991. "Assignment of the human prohibitin gene (PHB) to chromosome 17 and identification of a DNA polymorphism," Genomics 11:228-230). In both human and rat, the prohibitin gene has six introns and seven exons (Altus, et al. 1995. "Regions of evolutionary conservation between rat and human prohibitin-encoding genes," Gene 158:291-294) and produces two transcripts, one apparently 1.2 and the other 1.9 kb in length, as defined by Northern blotting experiments (Nuell, et al. 1991. Mol Cell Bio 11:1372-1381; Jupe, et al. 1995. Exp Cell Res 218:577-580; Jupe et al. 1996. Exp Cell Res 224:128-135; and Jupe, et al. 1996. Cell Growth and Differentiation 7:871-878). Both transcripts code for the same 30,000 Dalton protein, and in mammalian tissues, the level of protein expression generally parallels the level of total message. (Nuell, et al. 1991. Mol Cell Bio 11:1372-1381; Jupe, et al. 1995. Exp Cell Res 218:577-580; Jupe et al. 1996. Exp Cell Res 224:128-135; Jupe, et al. 1996. Cell Growth and Differentiation 7:871-878; White, et al. 1991. Genomics 11:228-230; Altus, et al. 1995. Gene 158:291-294; and McClung, et al. 1995. "Prohibitin: potential role in senescence, development, and tumor suppression," Exp Gerontol 30:99-124). In addition to rat and human, prohibitin has been cloned from mouse (Terashima, et al. 1994. "The IgM antigen receptor of B lymphocytesis associated with prohibitin and a prohibitin-related protein," EMBO J 13:3782-3792), yeast (Franklin, D.S. and Jazwinski, S.M. 1993. "A yeast homolog of the rat prohibitin gene is differentially expressed and determines longevity in Saccharomyces cerevisiae, J Cell Biochem Suppl 17D: 159), *Drosophila* (Eveleth, D.D.J. and Marsh, J.L. 1986. "Sequence and expression of the Cc gene, a member of the dopa decarboxylase gene cluster of Drosophila: possible translational regulation," Nucleic Acids Res, 14:6169-6183), and *Pneumocystis carinii* (Narasimhan, et al. 1997. "Prohibitin, a putative negative control element present in Pneumocystis carinii," Infection and Immunity 65:5125-5130). The protein is highly conserved throughout evolution, and the deduced amino acid sequences of human and rat prohibitin are identical except for one conservative amino acid change. (McClung, et al. 1995. Exp Gerontol 30:99-124; and Sato, et al. 1992. "The human prohibitin gene located on chromosome 17q21 is mutated in sporadic breast cancer," Cancer Res 52:1643-1646). The majority of the protein is localized to the mitochondria, and roles in diverse processes such as cellular aging in yeast (Jazwinski, S.M. 1996. "Longevity, genes, and aging," Science 273:54-59; and Coates, et al. 1997. "The prohibitin family of proteins regulate replicative lifespan," Current Biology 7:R607-R610), development and viability in *Drosophila* (Eveleth, D.D.J. and Marsh, J.L. 1986. Nucleic Acids Res, 14:6169-6183), and granulosa cell proliferation in mammals (Thompson, et al. 1997. "Steroidogenic acute regulatory (StAR) protein (p25) and prohibitin (p28) from cultured rat ovarian granulosa cells," J Reproduct Fertility 109:337-348) have been reported. In yeast and *Pneumocystis,* prohibitin protein has been shown to have a possible role in the *ras* signalling pathway. (Narasimhan, et al. 1997. Infection and Immunity 65:5125-5130; and Jazwinski, S.M. 1996. Science 273:54-59). Prohibitin has been shown to interact with retinoblastoma tumor suppressor proteins (Rb) in vivo and in vitro and was effective in repressing E2F-mediated transcription, while a prohibitin mutant could not bind to Rb, repress E2F activity, or inhibit cell proliferation (Wang, et 1. 1999. "Prohibitin, a potential tumor suppressor, interacts with RB and regulates E2F function," Oncogene 18:3501-3510).

Prohibitin gene structure and function have been examined in eleven immortalized human cell lines which have been classified into four complementation groups (A-D). (Jupe, et al. 1995. Exp Cell Res 218:577-580; and Jupe et al. 1996. Exp Cell Res 224:128-135). Human breast cancer cell lines have also been examined because the gene is located at 17q21, a chromosomal region that frequently undergoes loss of heterozygosity (LOH) in sporadic and familial breast cancers. (Nagai, et al. 1994. "Detailed deletion mapping of chromosome segment 17q12-21 in sporadic breast tumors," Genes, Chromosomes, and Cancer 11:58-62). Cell proliferation assays performed following the introduction of full length (1.9 kb) wild type prohibitin RNA showed that normal human diploid fibroblasts (HDF), 75% of breast cancer cell lines examined, and all four Group B cell lines (but no cell lines from any of the other groups) were inhibited at the G1-S transition in the cell cycle. (Jupe, et al. 1995. Exp Cell Res 218:577-580; Jupe et al. 1996. Exp Cell Res 224:128-135; and Jupe, et al. 1996. Cell Growth and Differentiation 7:871-878). Surprisingly, sequence analysis of the prohibitin gene showed that the 3' UTR from prohibitin sensitive cancer cell lines differed from wild type at one or more bases, while the 3' UTR from insensitive lines exhibited the wild type sequence. There were no coding region sequence alterations in any of the cell lines. (Jupe, et al. 1995. Exp Cell Res 218:577-580; Jupe et al. 1996. Exp Cell Res 224:128-135; and Jupe, et al. 1996. Cell Growth and Differentiation 7:871-878). These findings suggest that the loss of growth control in the cancer cell is due to mutations in the prohibitin 3' UTR.

International Application No. WO 96/40919 published December 19, 1996, disclosed that mutations in the 3' UTR of the B type allele are diagnostic for increased susceptibility to breast cancer and that reintroduction of either a portion or entire normal 3' UTR of the prohibitin gene into early stage tumors can be employed as a therapeutic agent for treatment of cancer.

International Application No. WO 98/20167 published May 14, 1998, disclosed a method for determining a patient's susceptibility to breast cancer by identifying the patient's germline genotype at position 729 in the prohibitin 3' UTR (SEQ ID NO:2) from either genomic DNA or RNA transcribed from genomic DNA which contains the 3' UTR of the prohibitin gene.

International Application No. WO 99/24614 published May 20, 1999, disclosed a method for determining a patient's susceptibility to other types of cancer besides breast cancer (e.g., prostate or ovarian cancer) by identifying the patient's germline genotype at position 729 in the prohibitin 3' UTR (SEQ ID NO:2) from either genomic DNA or RNA transcribed from genomic DNA which contains the 3' UTR of the prohibitin gene.

U.S. Patent No. 5,776,738 issued July 7, 1998 and U.S. Patent No. 5,922,852 issued July 13, 1999 disclosed a purified nucleic acid fragment consisting of a portion of the 3' UTR region of the prohibitin gene which can be used in determining a patient's susceptibility to breast cancer and other cancers.

It has now been found that the introduction of single stranded oligonucleotides, preferably DNA from the 3' UTR of wild-type prohibitin or RNA transcribed therefrom, or synthetically manufactured oligonucleotides of like sequence, most preferably RNA transcribed from the wild type prohibitin 3' UTR, into three breast cancer cell lines leads to arrested cell proliferation, while RNA transcribed from mutated prohibitin 3' UTR has no antiproliferative activity. Thus, growth control is reestablished when wild-type prohibitin RNA is introduced. Whereas cellular proliferation assays have previously demonstrated cell cycle arrests following the introduction of genes coding for p53 or p21 cyclin dependent kinase inhibitor (i.e., WAF1, SDI1, CAP20 or CIPI) and subsequent production of functional protein, it has now been found that wild type RNAs transcribed from portions of the prohibitin 3' UTR (or other oligonucleotides as described herein) can be directly administered as therapeutic agents against cancer, thus bypassing the requirements of the more "traditional" gene therapy approaches where a protein-producing gene is introduced into the cell or chromosome.

### SUMMARY OF THE INVENTION

The present invention relates to polynucleotides derived from the sequences of a 3' untranslated region (3' UTR) of a prohibitin gene, namely to a single-stranded polynucleotide as defined in claim 1. In one aspect, the polynucleotide is ribonucleic acid transcribed from a portion of the 3' untranslated region of the prohibitin gene which has tumor suppressor activity in animals, preferably mammals. A preferred ribonucleic acid is a transcript from position 1566 to 1803 of SEQ ID NO:1, the final 238 nucleotides of the 3' end of the 3' untranslated region of the prohibitin gene. SEQ ID NO:3 depicts this preferred sequence preceded by a T7 promotor.

Single stranded DNA derived from the 3' UTR which mimics the three dimensional conformation of the RNA described above also comprise oligonucleotides of the present invention. It is comprised of bases from position 1566-1803 of SEQ ID NO:1.

The present invention also includes DNA from which the RNA transcripts are transcribed. In addition, the RNA and DNA can be made synthetically through methods known in the art by using the sequence information provided herein.

In another aspect, the present invention is a pharmaceutical preparation adapted for administration to obtain an anticancer effect, comprising a proliferation-inhibiting amount of active isolated oligonucleotides. In one aspect, an isolated ribonucleic acid as defined above can be used in a pharmaceutical preparation. The preparation may include any of the RNAs or DNAs described above. The pharmaceutical preparation comprising a ribonucleic acid can be administered so that it directly contacts a tumor. The preferred method is by intra-tumoral injection. The dosage amount for directly contacting a tumor is at from about 2 micrograms to about 120 micrograms per cubic centimeter of tumor. The volume of the tumor can be estimated by ultrasonic visualization or other methods for estimation known in the art of medicine. The pharmaceutical preparation can also be administered systemically. The dosage amount for systemic administration by continuous infusion (iv) is at from about 0.5 milligrams to about 200 milligrams per kilogram body weight per day for one to fifteen days. More preferably the dosage is from 1-100 mg/kg/day.

In another aspect, single stranded DNA oligonucleotides as described above may be used.

In yet another aspect, the present invention is the use of the said polynucleotides in the manufacture of a pharmaceutical for the treatment of cancer. In one aspect the invention relates to compositions comprising the polynucleotides described above for use in human or veterinary medicine.

### BRIEF DESCRIPTION OF THE DRAWINGS

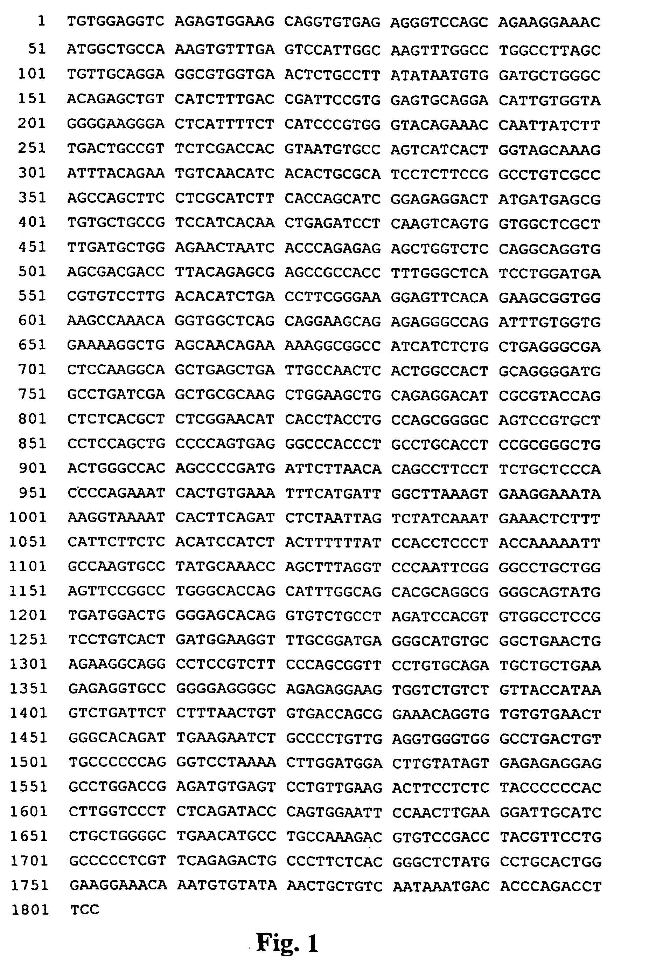

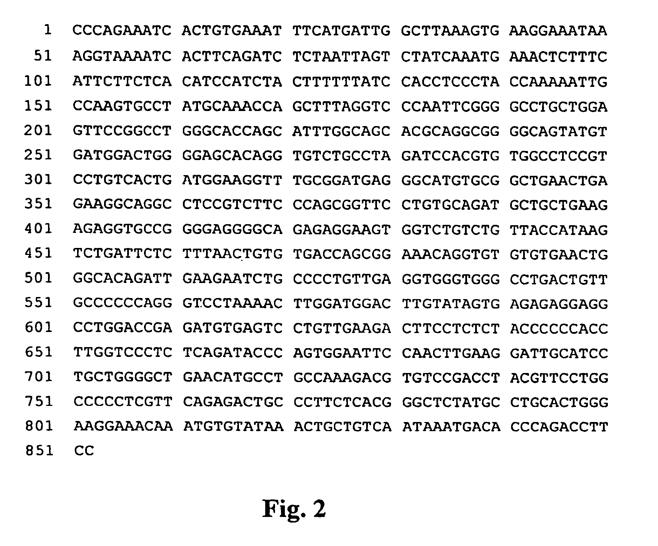

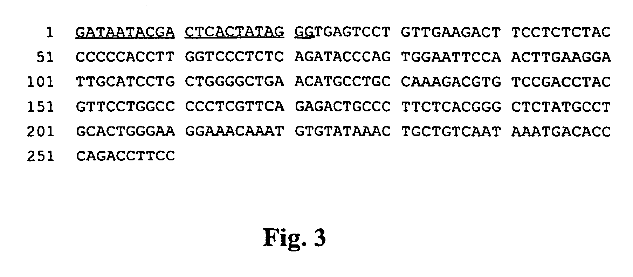

Fig. 1 (SEQ ID NO: 1) shows the sequence (5'→3') of the sense strand of a human prohibitin cDNA of 1803 bases encoding the RNA referred to as the 1.9kb transcript detected by Northern blotting experiments. The protein coding region of the transcript ends at position 866. The transcript historically referred to as 1.2 kb ends at position 1026. Thus, the sequence from position 1027 to 1803 is 3' UTR that is unique to the longer transcript. An 852 base transcript that overlaps by about 75 bases with the 3' UTR of the 1.2kb transcript, begins at position 952 and ends at position 1803 of the 1.9kb transcript (SEQ ID NO:1). This was the first 3' UTR specific fragment shown to have antiproliferative activity (Fig.2). The antiproliferative activity has now been further localized to the final 238 bases of the 3' UTR (Fig. 3; SEQ ID NO:3). This subfragment represents a portion of the 3' UTR unique to the longer transcript.
Fig. 2 depicts the DNA sequence of the sense strand (5'→3') of the region coding for the 852 base transcript of wild type prohibitin 3' UTR (SEQ. ID NO:2).
Fig. 3 depicts the DNA sequence of the sense strand (5'→3') of the region coding for a 238 base transcript (3' end of 1.9kb wild type prohibitin 3' UTR) (SEQ ID NO:3). The T7 primer sequence used to produce the RNA from PCR products is underlined at the 5' end Transcription of the RNA from the T7 promoter contained on a PCR product begins at the first G of the GGG at the end of the promoter. This produces an RNA with 238 bases of prohibitin sequence and two additional Gs on the 5' end.

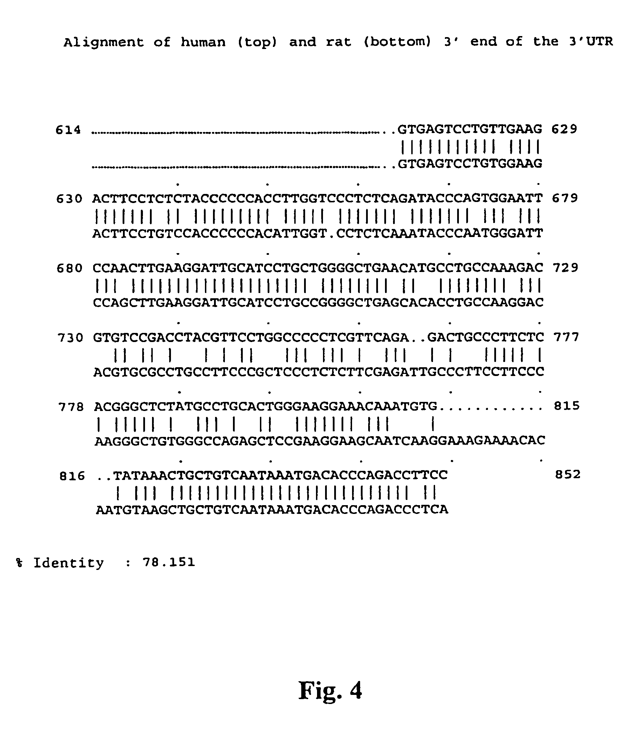

Fig. 4 depicts the alignment of the 3' end of the 3' UTR of the wild type human (top) and rat (SEQ ID NO:4) (bottom) prohibitin genes, showing 78 % identity as determined by the GAP program in the University of Wisconsin GCG package.

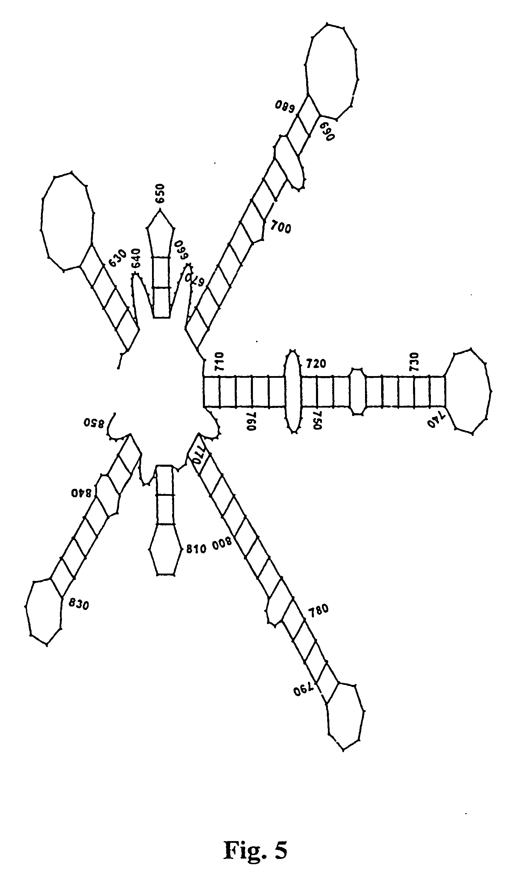

Fig. 5 depicts the predicted secondary structure formed by the wild type therapeutic RNA. The model was generated by the MFOLD program and the graphic representation was generated with the PLOTFOLD program. Both programs were run using the University of Wisconsin GCG package.

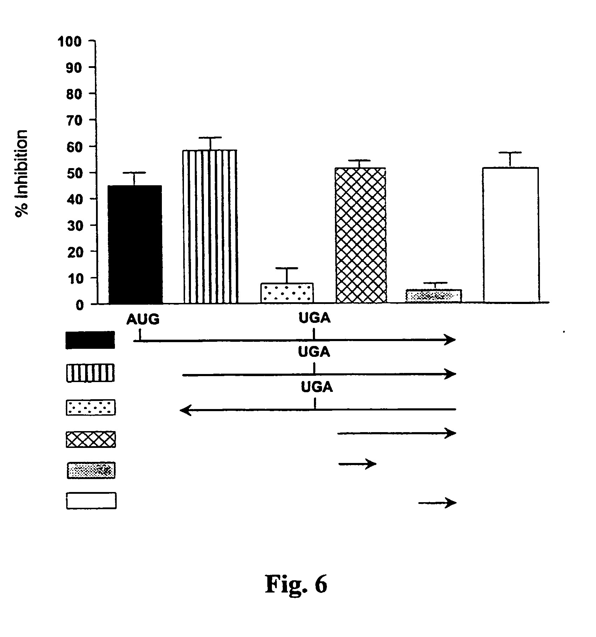

Fig. 6 depicts localization of the antiproliferative activity of prohibitin RNA into a subfragment of the 3' UTR. Schematics of the transcripts used to map the activity to the 3' end of the 3' UTR are shown at the bottom of the figure and coded as: closed bar = full length, 1.9 kb (Fig. 1); stripe bar = truncated transcript, missing the start site as well as the codons for the first 40 amino acids of prohibitin protein; dotted bar = antisense of the truncated transcript; cross hatched bar = the 852 base 3' UTR specific transcript (Fig. 2); partially shaded (gray) bar = the first 238 bases of the 852 base 3' UTR; open bar = the last 238 bases from the 3' end of the 3' UTR (Fig. 3). The location of the start (AUG) and termination (UGA) codons are shown. The values reported are the mean inhibition of the percentage of cells in the S phase of the cell cycle calculated from three separate experiments ± SEM. Normal HDFs were micro-injected with the indicated RNAs in all of the experiments.

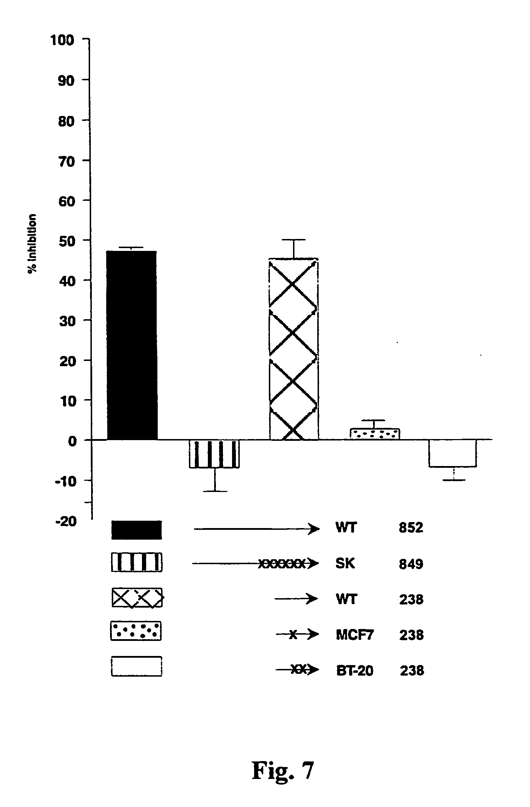

Fig. 7 depicts the effect on cell cycle progression by microinjection of mutated prohibitin 3' UTR transcripts into normal HDFs. Schematics of the 3' UTR transcripts used in each experiment are shown at the bottom of the figure and are coded as: closed bar = wild type, 852 base transcript; vertical stripe bar = SK-BR-3 heavily mutated, 849 base transcript; cross-hatched bar = the 3' end of the wild type 3' UTR transcript, 238 base transcript; dotted bar = the 3' end of the MCF7 3' UTR transcript, 238 base transcript; open bar = the 3' end of the BT-20 3' UTR transcript, 238 base transcript. The "x" designations indicate the approximate locations of the mutations. The values reported are the mean inhibition of the percentage of cells in the S phase of the cell cycle calculated from three separate experiments ± SEM. Normal HDFs were micro-injected with the indicated RNAs in all of the experiments. The bars with a negative value represent a slight stimulation of cell proliferation.

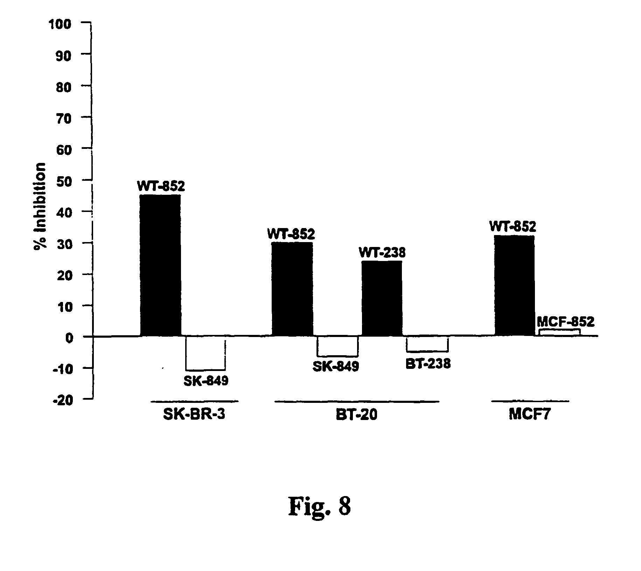

Fig. 8 depicts the effect on cell cycle progression by microinjection of wild type and mutated 3' UTR transcripts into breast cancer cell lines. Wild type (solid bar) or mutated (open bar) transcripts were microinjected into different breast cancer cell lines (shown at bottom of figure). Each bar shows the level of inhibition of the percentage of cells in the S phase of the cell cycle observed for one experiment (200-400 cells tested) and is labeled with the microinjected transcript. The bars with a negative value represent a slight stimulation of cell proliferation.

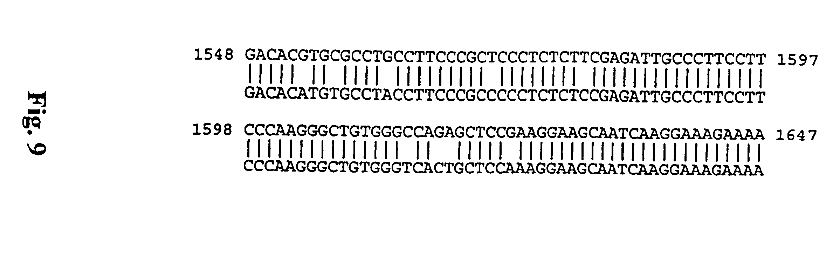

Fig. 9 depicts sequence alignment of a portion of the wild type and mutated DNA sequences coding for rat prohibitin 3' UTR. The wild type rat sequence (top) has been previously published, (Nuell, et al. 1991. Mol Cell Biol 11:1372-1381), while the mutated molecule was cloned from 7, 12-dimethylbenz(a)anthracene (DMBA)-induced rat mammary tumor tissue. There are nine mutations over this 100 base region.

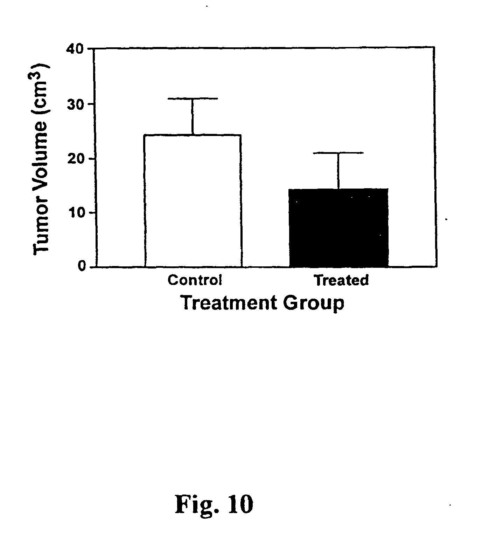

Fig. 10 depicts the effect of injection of the wild type human 238 base 3' UTR RNA (Fig. 3 and SEQ ID NO:3) on tumor growth in vivo in Example 1. The bars show mean tumor size and standard deviation on Day 30 following tumor injection, which was equal to 20 days after initial treatment. In the treated group, palpable primary tumors were injected with 10 micrograms of 238 base prohibitin 3' UTR RNA on Days 10 and 15 for a total of two doses. The control group was treated with isotonic saline carrier at the same time. The values are significantly different as determined by student's t-test (p≤0.01).

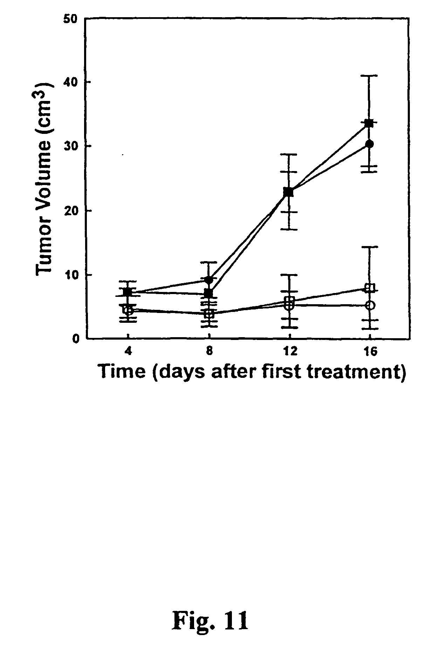

Fig. 11 depicts the effect of prohibitin 3' UTR RNA therapy on treatment groups from Example 2. Tumor volumes measured at four day intervals are shown for groups as: closed circle=saline control; closed square=5' end of the 3' UTR control; open square=low dose wild type 3' end of the 3' UTR (10 micrograms of RNA per injection); and open circle=high dose wild type 3' end of the 3' UTR (20 micrograms of RNA per injection). The mean values with standard deviations are plotted.

### DETAILED DESCRIPTION

Novel oligonucleotides derived from the 3' untranslated region (3' UTR) of the prohibitin gene can be used to reduce proliferation of cancer cells, regress tumors, and treat cancer. For example, RNAs derived from the non-coding prohibitin 3' UTR or single-stranded sense DNAs of the non-coding prohibitin 3' UTR which mimic the three-dimensional conformation of the RNA derived from the non-coding wild type prohibitin 3' UTR can be applied to cells or tumors or administered to patients in need of treatment in order to provide these aforementioned benefits. More specifically, the novel polynucleotides are derived from the human non-coding 3' UTR of the wild type prohibitin 1.9 kb transcript (Fig. 1 and SEQ ID NO:1). In the 1.9 kb transcript, the protein coding region ends at base number 866. The 1.9 kb transcript contains about 777 bases of unique 3' UTR beginning at position 1027 (Fig. 1 and SEQ ID NO:1) (Jupe et al. 1996. Exp Cell Res 224:128-135). The polynucleotide of the invention is single-stranded and comprises a portion of 238 bases of the 3' UTR of a prohibition gene defined by SEQ ID NO: 1, and lacks prohibitin sequences 5' to position 1566 of SEQ ID NO:1.

Also described are animal variants for the treatment of cancer in humans and other animals, such as oligonucleotides derived from the 3' UTR of the prohibitin gene from a non-human animal which have 50% or greater homology or more preferably 70% or greater homology with the 3' UTR of the human prohibitin gene. More specifically, as illustrated in the alignment of the wild type human and 3' end of the prohibitin 3' UTR given in Fig. 4, the 3' end of the 3' UTR of the rat prohibitin gene has 78% homology with the 3' end of the 3' UTR of the human prohibitin gene, and, therefore, oligonucleotides comprising the 3' end of the rat 3' UTR can be used to treat cancer in humans and other animals. Experimentally the homology can be defined by hybridization of the human sequence in a salt solution to other animal DNAs (or RNAs) bound to a nylon membrane. The stringency of the reaction is determined by the melting temperature Tₘ being defined by the formula Tₘ=81.5+0.41(G+C) + 16.6 log [Na⁺] - 0.63(% formamide) -[300+2000 [Na⁺]÷ d]: where G + C is the % guanosine and cytosine in the probe of known sequence, [Na⁺] is the molarity of Na ⁺ or equivalent monovalent action and d is the length of the hybridized duplex in nucleotides. Conditions of 40-50° below Tₘ can be used for very distant homologs. Screening at 30-35° below Tₘ is usually preferred to identify more closely related homologs.

### Oligonucleotide Therapy

The data shown below demonstrate that a short fragment of a prohibitin 3' UTR RNA transcript functions as a regulatory RNA with tumor suppressor activity and is able to effectively control breast tumor cellular proliferation in vivo when administered as a therapeutic. These results indicate the effectiveness of a direct prohibitin 3' UTR RNA therapy for breast cancer in humans and other animals. The presence of mutations in the prohibitin 3' UTR have also been found in other types of cancer and wild type prohibitin RNA can inhibit cell cycle progression in cell lives derived from other types of tumors. Accordingly, direct prohibitin 3' UTR polynucleotide therapy can also be used in the treatment of other types of cancer.

Prohibitin 3' UTR-derived polynucleotides can be administered either directly into solid tumors or systemically. If administered prior to the surgical removal of the tumor, shrinkage of the tumor may be sufficient so that surgery is no longer necessary thereby avoiding the possible undesirable release of tumor cells into the circulation as a complication of surgery. The preferred initial dose for polynucleotides injected directly into a solid tumor in the treatment of cancer is from about 2 micrograms to about 120 micrograms per cubic centimeter tumor, or for systemic treatment 0.5 mg/kg/day to 200 mg/kg/day for one to fourteen days. The more preferred dose for polynucleotides administered systemically in treatment of cancer is from about 1 to about 100 milligrams per kilogram body weight/day. The treatment can be repeated as deemed desirable.

The polynucleotides of the present invention can be administered by any means which transmit a proliferation-inhibiting amount of the polynucleotide into malignant cells. Other methods of administration include direct delivery to the liver via the hepatic portal system, to the nervous system by intra-thecal injection and to the lungs by inhalation. The carrier used can be isotonic saline or more preferably, cationic or anionic liposomes. An alternative method is to insert the polynucleotide preparation into tissue surrounding the tumor, at which time the oligonucleotides migrate into the malignant cells. The polynucleotide preparation can be injected by syringe directly into accessible tumors or tissue surrounding accessible tumors, either intraveneously, intramuscularly, intraperitoneally, subcutaneously, or topically. Administration to mucosal surfaces by way of creams, sprays, liquids and swabs is also anticipated. The composition can be administered to the lungs by means of a pulmonary inhaler. Oral administration may also be used.

Prohibitin 3' UTR-derived polynucleotides are systemically delivered either intraveneously, intramuscularly, intraperitoneally, or subcutaneously; however, other methods known in the art are within the scope of the present invention. The composition may be encapsulated or incorporated into a vehicle used in the pharmaceutical art for delivery such as, for example, liposomes.

In addition, for either direct or systemic treatment, the polynucleotides can be administered in an expression vector. Expression vectors suitable for polynucleotides of the present invention include viral vectors, plasmid vectors, and other vehicles known in the art that have been manipulated by insertion of sequences encoding the prohibitin 3' UTR. Preferable expression vectors include eukaryotic viral vectors, for example, bovine papilloma virus, adenovirus, and adeno-associated virus. The expression vector contains a promoter that facilitates the efficient, preferably constitutive, production of the oligonucleotides of interest in the patient. Most preferred for constitutive activity is the cytomegalo virus (CMV) promoter. Promoters specific for the targeted malignant cell type are advantageous, i.e., mammary tumor virus promoter for treatment of breast cancer. Preferable promoters specific for breast tissue include those for milk proteins, e.g. β-casein, β-lactoglobin, or whey acidic proteins.

Alternatively, the polynucleotide can be combined with a pharmaceutically acceptable carrier or excipient which is selected based on the method of administration. Excipients can include fillers, extenders, binders, disintegrants, surface-active agents, or lubricants for use in a variety of dosage forms including suspensions, emulsions, solutions, creams, gels, salves, and liquid preparations for injection. Tablets, powders, granules, and capsules can be used for oral administration. Various carriers can be used for the delivery of the prohibitin 3' UTR-derived polynucleotides. Exemplary carriers include saline, the cationic liposome, (N-[1-(2,3-dioleoyloxy)propyl]-N,N,N-tri-methylammonium methylsulfate) (DOTAP, Boehringer Mannheim, Indianapolis, IN), and other cationic or anionic liposomes.

Moreover, the success of the direct injection of the prohibitin RNA into tumors indicated that the structure of the RNA has some inherent stability and nuclease resistance, possibly due to its propensity to form secondary structure (Fig. 5). Migration of the prohibitin RNA on native gels was consistent with the molecule being essentially double-stranded. In addition, modifications to the oligonucleotides of the present invention which improve nuclease resistance and molecular stability can be used in cancer treatment. For example, the addition of a 5' terminal 7-methyl guanosine cap or a poly-A tail confers greater stability to RNA.

The methods for making the prohibitin 3' UTR RNAs of the present invention and methods of treatment with the RNAs are described below. Although the present invention has been presented herein with respect to certain descriptions and examples, one of ordinary skill in the art will appreciate that certain modifications can be made to the present invention without departing from its true spirit and scope.

### Localization of Antiproliferative Activity

Localization of the antiproliferative activity into a subfragment of the prohibitin 3' UTR was first performed according to the microinjection assay procedure given in Example 4. The full-length 1.9-kilobase (kb) cDNA of the prohibitin 3' UTR and several truncated cDNAs cloned into either pBSIISK+ (Stratagene, La Jolla, CA) or pCRII (Invitrogen, San Diego, CA) were used to synthesize RNA transcripts in vitro using the mMESSAGE mMACHINE (Ambion, Inc., Austin, TX). Truncated transcripts were synthesized in both the sense and antisense orientation from a clone missing translational signals as well as the AUG start site and the region coding for the first 40 amino acids of the prohibitin protein. The 852 base 3'UTR transcripts beginning approximately 80 bases downstream from the UGA stop codon were synthesized from a 3' UTR clone with wild-type sequence. Transcripts were also synthesized from the first 238 bases of the 852 fragment of the 3' UTR and from the final 238 bases of the 3' UTR.

The antiproliferative activity was localized in the wild type prohibitin transcript by comparing the ability of full length and truncated transcripts to inhibit cell cycle progression when microinjected into normal HDFs (Fig. 6). A full length sense transcript truncated at the 5' end, the 852 base non-coding 3' UTR alone (Fig. 2 and SEQ ID NO:2), and the final 238 bases of the 3' UTR (Fig. 3 and SEQ ID NO:3) all exhibited antiproliferative activity comparable to that of the full length 1.9 kb transcript. The truncated antisense transcript and the 238 bases from the 5' end of the 852 base 3' UTR both had little or no antiproliferative activity. Thus, the antiproliferative activity mapped to the final 238 bases of the 3' UTR, indicating that mutation in this region leads to loss of antiproliferative activity.

### Antiproliferative Activity of Mutated Prohibitin Transcripts in HDFs and Breast Cancer Cell Lines

Antiproliferative activity of prohibitin RNA corresponding to those isolated from three breast cancer cell lines (BT-20, MCF7, and SK-BR-3) was performed according to the microinjection assay procedure given in Example 4. The RNA transcripts were produced according to the method given in Jupe, et al. 1996. Exp Cell Res, 224:128-135. The mutated RNAs corresponding to those from three breast cancer cell lines (Table I) were unable to inhibit cell cycle progression in normal HDF (Fig. 7). Even the single cytosine (C-729) to thymine (T-729) transition at UTR-729 which represents an allelic polymorphism was sufficient to eliminate activity from the final 238 bases of the 3' UTR in MCF7. However, BT-20, which is mutated at two other sites but has C-729, also lacked antiproliferative activity, demonstrating that multiple alterations can cause a loss of the 3' UTR's antiproliferative activity. Another experiment illustrated in Fig. 8 showed that in breast cancer cell lines with mutations in the final 238 bases, the wild type 852 and final 238 base RNAs both had antiproliferative activity while mutated ones were inactive, just as they were in HDF. These data show that the antiproliferative activity is localized to the 3' end of the 3' UTR, the most frequently mutated region in breast cancer cell lines (Jupe, et al. 1996. Cell Growth and Differentiation 7:871-878) and breast tumors.

### Antiproliferative Activity of Prohibitin 3' UTR RNA in Animal Models

RNA prepared by in vitro transcription of a 238 base fragment of the prohibitin 3' UTR (Fig. 3 and SEQ ID NO:3) has been used to treat rat mammary tumors. The RNA was administered by direct injection into a recently emerged palpable tumor. The initial experiments were done with the RNA resuspended in isotonic saline for administration. Using this delivery method, tumors have been completely cured on 50% of treated animals. Modification of the therapeutic RNA by addition of a 5'-terminal-7 methyl guanosine cap, known to stabilize eukaryotic RNA, along with delivery of the RNA complexed with the cationic-liposome DOTAP (N-[1-(2,3-dioleoyloxy)propyl]-N,N,N-tri-methylammonium methylsulfate) led to improved cure rates. The long term survival rate under these conditions was increased to 75%. The successfully treated animals underwent regression of both the treated primary tumor as well as untreated distant metastases.

**Table I: Prohibitin 3' UTR Mutations in Three Breast Cancer Cell Lines^{a}**

| Cell Line | Positions of Base Changes | Base Changes |
|---|---|---|
| BT-20 | 758 | G to A |
| | 814 | T to C |
| MCF7 | 236 | G to A |
| | 729 | C to T |
| SK-BR-3 | 691 | G to T |
| | 696 | C to A |
| | 702 | G to T |
| | 711 | GtoA |
| | 729 | C to T |
| | 734 | C to A |
| | 736 | G to A |
| | 737 | A to T |
| | 741 | AtoC |
| | 742 | C to T |
| | 752 | C to T |
| | 753 | C to T |
| | 757 | C to T |
| | 771 | C to T |
| | 778 | A to C |
| | 779 | C to A |
| | 787 | AtoG |
| | 794 | C to T |
| | 795 | A to G |
| | 798 | G to T |
| | 799 | G to T |
| | 802 | ΔA |
| | 803 | AG |
| | 804 | AG |
| | 808 | C to T |
| | 810 | A to G |
| | 813 | G to C |
| | 825 | C to T |
| | 829 | C to T |

| | | |
|---|---|---|
| ^{a} The prohibitin sequence of breast cancer cell lines was compared to the wild type sequence from normal HDFs (GenBank Acc. #U49725). Two base changes are present in BT-20 and MCF7, but 26 base changes and a 3 base deletion at 802-804 are detected in SK-BR-3. | | |

RNA Production. Prohibitin RNA was prepared by in vitro transcription using either the mMESSAGE mMACHINE™ or the MEGAScript™ transcription kit (Ambion). The mMESSAGE mMACHINE includes a cap analog (m⁷G(5')ppp(5')G) in the reaction which incorporates a 5' terminal G into the transcript. This 7-methyl guanosine cap structure at the 5' end of the eukaryotic RNAs confers greater stability to the molecule. Transcription templates used were either a cloned and sequenced plasmid containing the wild type human (or rat) prohibitin 3' UTR or a PCR product subfragment synthesized from the cloned plasmid using prohibitin primers with minimal phage promoters (T7 or SP6) attached (transcription-linked primers)(Fig. 3; SEQ ID NO:3). Following transcription, an aliquot of the reaction was assayed on denaturing agarose gels to confirm the integrity and estimate the yield of full length transcripts. The *in vitro* transcripts were digested with RNase-free DNasel. The RNA was then purified on 1.5% agarose gels, eluted from the gel, ethanol precipitated and resuspended in RNase-free water. Alternatively, after DNaseI digestion, the reaction was stopped by adding nuclease-free water and ammonium acetate. The mixture was then phenol/chloroform extracted, chloroform extracted and precipitated with isopropyl alcohol. The precipitated samples were resuspended in sterile, RNase free water. The concentration of RNA was determined spectrophotometrically.

Formulation in Isotonic Saline. The amount of RNA required to achieve the desired dose was reprecipitated using ammonium acetate and isopropanol and resuspended at a concentration of 0.5-1.0 microgram/microliter (µg/µl) in pyrogen-free isotonic saline (Baxter Healthcare Corp., Deerfield, IL, 0.9% sodium chloride) for administration. RNA for injection was prepared by further diluting 10 to 100 micrograms to a final volume of 100 -200 microliters of isotonic saline.

Formulation in DOTAP. The preferred method of RNA delivery involves the formation of a complex with N-[1-(2,3-Dioteoyloxy)propyl]-N,N,N,-trimethylammonium methylsulfate (DOTAP; Roche Molecular Biochemicals, Indianapolis, IN), a reagent commonly used for cationic liposome-mediated transfection of eukaryotic cells. The RNA/DOTAP complexes were formed in a HEPES-buffered saline (HBS) (1X HBS = 20 mM HEPES, 150 mM NaCl, pH 7.4) following the general guidelines provided in the manufacturer's instructions. The mixture for injection was prepared as follows for a 200 microliter delivery volume containing a dose of 20 micrograms of RNA. A 100 microliter DOTAP solution was prepared by adding 30 microliters of DOTAP stock ( 1 microgram/microliter) to 70 microliters of 1X HBS. A 100 microliter solution of RNA stock was prepared by adding 20 micrograms of RNA (typically 20 microliters) and 10 microliters of 10X HBS to 70 microliters of RNAase free water. The nucleic acid solution was transferred dropwise into the DOTAP solution and mixed by pipetting 5-10 times. The RNA/DOTAP mixture was incubated for at least 10 minutes at room temperature prior to injection.

Treatment. The RNA prepared for delivery in either isotonic saline or as a DOTAP complex was directly injected into palpable tumors using a 28 gauge, lcc insulin syringe. In the rat mammary tumors, 100 -200 microliters of solution were typically delivered. Tumor radius was determined by measuring the diameter at the point of greatest length (D₁) and width (D₂) with calipers and dividing by 2 to calculate radius R₁ and R₂ respectively. The volume in cubic centimeters is calculated by the formula 4/3 πR₁ ² R₂ where R₁ ≤ R₂. Volume of internal tumors may be estimated by ultrasound or X-ray techniques.

Production of Single-Stranded DNA. One method of producing single-stranded DNA of a chosen strand is a modification of the polymerase chain reaction (PCR) in which unequal concentrations of the two amplification primers are used (asymmetric PCR). The single stranded DNA produced by this method can be purified on native or denaturing gels. The single stranded DNA band is excised and prepared for use by organic extraction and ethanol precipitation, as described above for RNA. *See, e.g.,* Peter McCabe (1990) Production of Single-Stranded DNA by Asymmetric PCR. In: M.A. Innis, D.H. Gelfand, J.J. Sninsky, T.J. White,eds. PCR Protocols: A Guide to Methods and Applications. Academic Press San Diego, pp 76-91. The sequences are selected from those which would be appropriate for transcription to RNA, as described above, but since DNA can be used as the therapeutic agent, no transcription is required. Rather, the single-stranded DNA prepared is utilized instead of RNA transcripts in the methods for treatment described herein.

### Synthesized Oligonucleotides

DNA or RNA oligonucleotides can be made synthetically, i.e. by use of automatic chemical synthesis or other methods known or which become available in the art. The sequences for synthesis are selected from those described for transcripted RNA or DNA derived from a 3'UTR of a prohibitin gene.

### Example 1

The efficacy of prohibitin 3' UTR RNA therapy was determined by direct injection into palpable 7,12-dimethylbenz(a)anthracene (DMBA)-induced mammary tumors in rats. These are aggressively growing, transplantable, metastatic mammary tumors that overexpress p53 and are propagated in female Wistar-Furth rats. This rat mammary tumor was originally induced in a female Wistar-Furth rat after exposure to 40 mg of DMBA. (Kollmorgen, et al. 1983. "Influence of dietary fat and indomethacin on the growth of transplantable mammary tumors in rats," Cancer Res 43:4714-4719; and Kim, U. 1980. "Characteristics of metastasizing and non-metastasizing tumors and their interaction with the host immune system in the development of metastasis." In: Hellman, K., Hilgar, P., and Eccles, S. (eds), EORTC Metastasis Group International Conference on Clinical and Experimental Aspects of Metastasis, The Hague: Martinus Nijhoff Publisher, pp. 210-214.). The tumor was maintained by passage every 30 days in 21 day old female, Wistar- Furth rats (130-140 grams). Primary tumor was homogenized to a single cell suspension in Dulbecco's Modified Eagle's Medium (DMEM) with 50 µg/ml gentamycin and 7% newborn calf serum (1x10⁷ cells/ml). Each animal received 1x10⁶ cells delivered in 200 microliters, injected into the fat pad of the sixth mammary gland, which is adjacent to the right inguinal lymph node. Palpable primary tumors at the injection site were evident between Day 10-12, and metastases were found in lymph nodes, lung, and liver by 20-30 days post injection. It was found for the first time that the tumor overexpressed p53 and had a mutated prohibitin 3' UTR (Fig. 9).

The 238 base RNA from the 3' end of the prohibitin 3' UTR was prepared by in vitro transcription with T7 polymerase using the MEGASCRIPT™ Transcription kit (Ambion) from a cloned and sequenced plasmid. After transcription, an aliquot of the reaction was assayed on denaturing agarose gels to confirm the production of full length transcripts. The in vitro transcripts were treated twice with RNase-free DNase. The RNA was then purified on 1.5% agarose gels, eluted from the gel slice, ethanol precipitated and resuspended in RNase-free water. The concentration was determined spectrophotometrically, and samples reprecipitated and resuspended in sterile, pyrogen-free isotonic saline (Baxter Healthcare Corp.) for administration.

Direct injection of prohibitin 3' UTR transcripts, produced in vitro, into emerging palpable solid tumors at 9-12 days after passage significantly reduced both the growth rate and the final size of the tumors. The wild type, 238 base prohibitin 3' UTR RNA which demonstrated the ability to inhibit cell cycle progression in cell lines (Figs. 6-8) was used. Inhibition of cell proliferation was observed when a primary tumor was treated with a single injection of 10 micrograms of RNA. In a series of ten treated animals (receiving a dose of 10 micrograms of RNA per injection and two injections) and ten control animals (receiving the saline carrier), the mean size (volume in cubic centimeters) of treated primary tumors was 42% (range 20-80%) smaller than control primary tumors at 30 days after passage (Fig. 10). All of the animals from the control group died (average lifespan = 36 ± 4 days), while four of the treated animals regressed to an apparently disease-free state and were healthy at 120 days past tumor injection. They had undergone complete regression of both the metastases and primary tumor by Day 45, indicating that a systemic anti-tumor activity was induced.

### Example 2

A 238 base RNA from the 3' end of the wild type prohibitin 3' UTR was examined for antiproliferative activity upon injection into rat mammary tumors in Wistar-Furth rats. Antiproliferative activity was observed with the prohibitin wild type RNA, while a control RNA had no effect on tumor growth.

Groups of five animals each were treated utilizing three injections into the primary tumor. There were two control groups, one receiving saline and another receiving RNA from the 5' end of the 3' UTR which was the same control used in cell proliferation assays (Fig. 6). The two treatment groups were low dose (10 micrograms in 100 microliters) and high dose (20 micrograms in 100 microliters) wild type RNA from the 3' end of the 3' UTR. By Day 16 after treatment, both groups of controls had tumors with a mean volume of about 30 cubic centimeters, while tumors in the low dose wild type treatment group were only 8 cubic centimeters and those of the high dose group were at 5 cubic centimeters (Fig. 11). Of the ten treated animals in this experiment, six animals were still alive and tumor free at 120 days after tumor injection as opposed to no animals in the control group. These results showed that both the dose and frequency of delivery altered the treatment efficacy.

### Example 3

Four treated animals from Example 1 and six treated animals from Example 2 underwent complete regression. In general, the animals that underwent regression had primary tumors that grew slowly, but they developed left and right axillary metastases. At approximately Day 15 following the initial treatment, the primary tumor ceased to grow, and the metastases also began to shrink. By Day 25, palpable metastases were no longer present, and the primary tumors had reduced in volume from a peak of about 20-30 cubic centimeters to only about 10 cubic centimeters. The primary tumors disappeared, and complete healing was observed between Day 35-45. The cured animals from Examples 1 and 2 remained disease free at 120 days after the initial tumor injection, while the average survival time of control animals was only 36 days. No spontaneous remission was observed in untreated animals or animals treated with control RNA.

This mode of regression, indicating that the prohibitin treatment triggers the development of an antitumor immune response, has now been tested by rechallenging 50 cured animals (120 days after initial challenge) with a fresh inoculation of the DMBA-4 tumor. Only 2 of the re-challenged animals developed tumors and the remaining 48 remained tumor free at 60 days after re-challenge showing that a long term immunity against the tumor was induced following successful prohibitin RNA therapy.

### Example 4

The antiproliferative activity of prohibitin RNA was assayed following the microinjection of RNA transcripts (Nuell, et al. 1991. Mol Cell Biol 11:1372-1381). Cells were synchronized in early G1 with lovastatin (provided by A.W. Alberts of Merck, Sharp and Dohme Research Pharmaceuticals, Rahway, NJ) according to the method of Keyomarsi, et al. 1991. Cancer Res 51:3602-3609. Synchronized cells (200-300 per experiment) were microinjected with an RNA transcript at a concentration of 1 mg/ml using an Eppendorf Model 5242 microinjector. Microinjected cells were allowed to move through the cell cycle in the presence of [³H]thymidine, and inhibitory activity was calculated following autoradiography (Nuell, et al., Mol Cell Biol 11: 1372-1381). These experiments show the antiproliferative activity is localized to a 238 base region at the 3' end of the 3' UTR (Fig.6). Mutation in this region leads to loss of antiproliferative activity in both normal cells and breast cancer cells (Figs. 7 & 8).

### Example 5

In this trial, sixteen Wistar-Furth rats were divided into two groups of eight. The control group of animals received an RNA 200 bases in length transcribed from the 5' end of the Xenopus elongation factor 1-α gene (Ambion Inc., Austin, Texas). The treatment group received the 238 base RNA transcript from the 3' end of the prohibitin 3' UTR. Control and prohibitin RNAs were produced with 7-mG caps using the mMESSAGE mMACHINE transcription kit. Doses containing 20 micrograms of RNA each were delivered in DOTAP carrier by intratumoral injection from a syringe on days 12 and 17 after tumor inoculation. The capping of transcripts and delivery in DOTAP carrier produced rates of tumor regression similar to that seen in isotonic saline. However, a major improvement in survival percentage was observed. In this experiment by day 55, all of the control animals had died while seven of the treated animals were alive. Six of the prohibitin RNA treated animals (75%) were cured and remained tumor free at 120 days following tumor inoculation.

### Example 6

Patients with primary tumors and/or metastases can be treated with at least one prohibitin 3' UTR oligonucleotide of the present invention, preferably complexed with the carrier DOTAP. For patients with primary tumors, the oligonucleotide can be administered by direct injection into the primary tumor at from about 2 to about 120 micrograms per cubic centimeter tumor volume. For patients with metastases following surgical removal of the primary tumor, the oligonucleotide can be administered systemically at from about 0.5 to about 200 milligrams per kilogram body weight. An administration cycle can be from 1 to about 15 days. The administration cycle can be repeated multiple times. For patients with primary tumors and secondary metastases, the oligonucleotide can be administered by direct injection into the primary tumor at from about 2 to about 120 micrograms per cubic centimeter tumor volume followed by systemic administration at from about 0.5 to about 200 milligrams per kilogram body weight. Patient progress is monitored through methods known to medical community such as ultrasonic techniques to ascertain tumor size and status.

### Example 7

A patient diagnosed with breast cancer can be provided with oligonucleotide therapy according to the parameters provided in the above example prior to surgery. The administration of the oligonucleotides of the present invention can provide benefits of tumor regression and/or prevention of metastasis which could otherwise be prompted by the surgery as cells are disturbed in the mechanical process of tumor removal.

## Claims

1. A single-stranded polynucleotide comprising a portion of 238 bases of the 3' untranslated region of a prohibitin gene defined by SEQ ID NO:1, wherein said polynucleotide lacks prohibitin sequences 5' to position 1566 of SEQ ID NO:1.

2. The single-stranded polynucleotide of Claim 1, wherein said polynucleotide is RNA.

3. The single-stranded polynucleotide of Claim 2, wherein said RNA is made synthetically.

4. The single-stranded polynucleotide of Claim 1, wherein said polynucleotide is DNA.

5. The single-stranded polynucleotide of Claim 4 consisting of said portion of said region.

6. The single-stranded polynucleotide of Claim 4, wherein said DNA is made synthetically.

7. A vector comprising the polynucleotide of Claim 1.

8. A composition comprising the polynucleotide of Claim 1 for use in human or veterinary medicine.

9. The composition of Claim 8 further comprising isotonic saline.

10. The composition of Claim 8 further comprising DOTAP.

11. The composition of Claim 8, wherein said composition is a liposome composition.

12. Use of the polynucleotide of Claim 1, 2 or 4 in the manufacture of a pharmaceutical for the treatment of cancer.

## Patentansprüche

1. Ein einzel-strängiges Polynukleotid umfassend einen Bereich von 238 Basen der 3' nichttranslatierten Region von einem Prohibitin-Gen, das durch die SEQ ID NO:1 definiert ist, wobei dem Polynukleotid die Prohibitin-Sequenzen 5' bis zur Position 1566 der SEQ ID NO:1 fehlen.

2. Das einzel-strängige Polynukleotid nach Anspruch 1, wobei das Polynukleotid RNA ist.

3. Das einzel-strängige Polynukleotid nach Anspruch 2, wobei die RNA synthetisch hergestellt ist.

4. Das einzel-strängige Polynukleotid nach Anspruch 1, wobei das Polynukleotid DNA ist.

5. Das einzel-strängige Polynukleotid nach Anspruch 4 bestehend aus dem genannten Bereich der genannten Region.

6. Das einzel-strängige Polynukleotid nach Anspruch 4, wobei die DNA synthetisch hergestellt ist.

7. Ein Vektor umfassend das Polynukleotid nach Anspruch 1.

8. Eine Zusammensetzung umfassend das Polynukleotid nach Anspruch 1 für die Verwendung in Human- oder Veterinärmedizin.

9. Die Zusammensetzung nach Anspruch 8 ferner umfassend isotonische Salzlösung.

10. Die Zusammensetzung nach Anspruch 8 ferner umfassend DOTAP.

11. Die Zusammensetzung nach Anspruch 8 wobei die Zusammensetzung eine Liposom-Zusammensetzung ist.

12. Verwendung des Polynukleotid nach Anspruch 1, 2 oder 4 zur Herstellung eines Arzneimittels zur Behandlung von Krebs.

## Revendications

1. Polynucléotide simple-brin comprenant une portion de 238 bases de la région 3' non-traduite d'un gène de la prohibitine défini par la SEQ ID NO : 1, dans lequel ledit polynucléotide est dépourvu de séquences de la prohibitine en 5' de la position 1566 de la SEQ ID NO : 1.

2. Polynucléotide simple-brin selon la revendication 1, dans lequel ledit polynucléotide est un ARN.

3. Polynucléotide simple-brin selon la revendication 2, dans lequel ledit ARN est préparé par synthèse.

4. Polynucléotide simple-brin selon la revendication 1, dans lequel ledit polynucléotide est un ADN.

5. Polynucléotide simple-brin selon la revendication 4 consistant en ladit portion de ladite région.

6. Polynucléotide simple-brin selon la revendication 4, dans lequel ledit ADN est préparé par synthèse.

7. Vecteur comprenant le polynucléotide selon la revendication 1.

8. Composition comprenant le polynucléotide selon la revendication 1 pour une utilisation en médecine humaine ou vétérinaire.

9. Composition selon la revendication 8 comprenant en outre une solution saline isotonique.

10. Composition selon la revendication 8 comprenant en outre du DOTAP.

11. Composition selon la revendication 8, dans laquelle ladite composition est une composition liposomale.

12. Utilisation d'un polynucléotide selon la revendication 1, 2 ou 4 pour la fabrication d'un médicament pour le traitement du cancer.


### SEQUENCE LISTING

<110> Jupe, Eldon R.
<120> USE OF PROHIBITIN RNA IN TREATMENT OF BREAST CANCER
<130> 11146/10102
<140>
   <141>
<150> US 60/093,977
   <151> 1998-07-24
<160> 4
<170> PatentIn Ver. 2.0
<210> 1
   <211> 1803
   <212> DNA
   <213> Homo sapiens
<400> 1
   tgtggaggtc agagtggaag caggtgtgag agggtccagc agaaggaaac atggctgcca 60
   aagtgtttga gtccattggc aagtttggcc tggccttagc tgttgcagga ggcgtggtga 120
   actctgcctt atataatgtg gatgctgggc acagagctgt catctttgac cgattccgtg 180
   gagtgcagga cattgtggta ggggaaggga ctcattttct catcccgtgg gtacagaaac 240
   caattatctt tgactgccgt tctcgaccac gtaatgtgcc agtcatcact ggtagcaaag 300
   atttacagaa tgtcaacatc acactgcgca tcctcttccg gcctgtcgcc agccagcttc 360
   ctcgcatctt caccagcatc ggagaggact atgatgagcg tgtgctgccg tccatcacaa 420
   ctgagatcct caagtcagtg gtggctcgct ttgatgctgg agaactaatc acccagagag 480
   agctggtctc caggcaggtg agcgacgacc ttacagagcg agccgccacc tttgggctca 540
   tcctggatga cgtgtccttg acacatctga ccttcgggaa ggagttcaca gaagcggtgg 600
   aagccaaaca ggtggctcag caggaagcag agagggccag atttgtggtg gaaaaggctg 660
   agcaacagaa aaaggcggcc atcatctctg ctgagggcga ctccaaggca gctgagctga 720
   ttgccaactc actggccact gcaggggatg gcctgatcga gctgcgcaag ctggaagctg 780
   cagaggacat cgcgtaccag ctctcacgct ctcggaacat cacctacctg ccagcggggc 840
   agtccgtgct cctccagctg ccccagtgag ggcccaccct gcctgcacct ccgcgggctg 900
   actgggccac agccccgatg attcttaaca cagccttcct tctgctccca ccccagaaat 960
   cactgtgaaa tttcatgatt ggcttaaagt gaaggaaata aaggtaaaat cacttcagat 1020
   ctctaattag tctatcaaat gaaactcttt cattcttctc acatccatct acttttttat 1080
   ccacctccct accaaaaatt gccaagtgcc tatgcaaacc agctttaggt cccaattcgg 1140
   ggcctgctgg agttccggcc tgggcaccag catttggcag cacgcaggcg gggcagtatg 1200
   tgatggactg gggagcacag gtgtctgcct agatccacgt gtggcctccg tcctgtcact 1260
   gatggaaggt ttgcggatga gggcatgtgc ggctgaactg agaaggcagg cctccgtctt 1320
   cccagcggtt cctgtgcaga tgctgctgaa gagaggtgcc ggggaggggc agagaggaag 1380
   tggtctgtct gttaccataa gtctgattct ctttaactgt gtgaccagcg gaaacaggtg 1440
   tgtgtgaact gggcacagat tgaagaatct gcccctgttg aggtgggtgg gcctgactgt 1500
   tgccccccag ggtcctaaaa cttggatgga cttgtatagt gagagaggag gcctggaccg 1560
   agatgtgagt cctgttgaag acttcctctc taccccccac cttggtccct ctcagatacc 1620
   cagtggaatt ccaacttgaa ggattgcatc ctgctggggc tgaacatgcc tgccaaagac 1680
   gtgtccgacc tacgttcctg gccccctcgt tcagagactg cccttctcac gggctctatg 1740
   cctgcactgg gaaggaaaca aatgtgtata aactgctgtc aataaatgac acccagacct 1800
   tcc 1803
<210> 2
   <211> 852
   <212> DNA
   <213> Homo sapiens
<400> 2
   cccagaaatc actgtgaaat ttcatgattg gcttaaagtg aaggaaataa aggtaaaatc 60
   acttcagatc tctaattagt ctatcaaatg aaactctttc attcttctca catccatcta 120
   cttttttatc cacctcccta ccaaaaattg ccaagtgcct atgcaaacca gctttaggtc 180
   ccaattcggg gcctgctgga gttccggcct gggcaccagc atttggcagc acgcaggcgg 240
   ggcagtatgt gatggactgg ggagcacagg tgtctgccta gatccacgtg tggcctccgt 300
   cctgtcactg atggaaggtt tgcggatgag ggcatgtgcg gctgaactga gaaggcaggc 360
   ctccgtcttc ccagcggttc ctgtgcagat gctgctgaag agaggtgccg gggaggggca 420
   gagaggaagt ggtctgtctg ttaccataag tctgattctc tttaactgtg tgaccagcgg 480
   aaacaggtgt gtgtgaactg ggcacagatt gaagaatctg cccctgttga ggtgggtggg 540
   cctgactgtt gccccccagg gtcctaaaac ttggatggac ttgtatagtg agagaggagg 600
   cctggaccga gatgtgagtc ctgttgaaga cttcctctct accccccacc ttggtccctc 660
   tcagataccc agtggaattc caacttgaag gattgcatcc tgctggggct gaacatgcct 720
   gccaaagacg tgtccgacct acgttcctgg ccccctcgtt cagagactgc ccttctcacg 780
   ggctctatgc ctgcactggg aaggaaacaa atgtgtataa actgctgtca ataaatgaca 840
   cccagacctt cc                                                     852
<210> 3
   <211> 260
   <212> DNA
   <213> Homo sapiens
<220>
   <221> promoter
   <222> (1)..(21)
   <223> T7 promoter sequence
<400> 3
   gataatacga ctcactatag ggtgagtcct gttgaagact tcctctctac cccccacctt 60
   ggtccctctc agatacccag tggaattcca acttgaagga ttgcatcctg ctggggctga 120
   acatgcctgc caaagacgtg tccgacctac gttcctggcc ccctcgttca gagactgccc 180
   ttctcacggg ctctatgcct gcactgggaa ggaaacaaat gtgtataaac tgctgtcaat 240
   aaatgacacc cagaccttcc                                             260
<210> 4
   <211> 254
   <212> DNA
   <213> Rattus sp.
<400> 4
   gtgagtcctg tggaagactt cctgtccacc ccccacattg gtcctctcaa atacccaatg 60
   ggattccagc ttgaaggatt gcatcctgcc ggggctgagc acacctgcca aggacacgtg 120
   cgcctgcctt cccgctccct ctcttcgaga ttgcccttcc ttcccaaggg ctgtgggcca 180
   gagctccgaa ggaagcaatc aaggaaagaa aacacaatgt aagctgctgt caataaatga 240
   cacccagacc ctca                                                   254